(19)

Europäisches
Patentamt
European
Patent Office
Office européen
des brevets

(11)  **EP 3 499 386 A1**

(12)  **EUROPEAN PATENT APPLICATION**

(43) Date of publication:
**19.06.2019  Bulletin 2019/25**

(51) Int Cl.:
**G06F 17/30** (2006.01)          **G06F 19/00** (2018.01)
**G16H 50/20** (2018.01)

(21) Application number: **17207148.2**

(22) Date of filing: **13.12.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **FUJITSU LIMITED**
**Kanagawa 211-8588 (JP)**

(72) Inventor: **HU, Bo**
**Winchester, Hampshire SO23 7DT (GB)**

(74) Representative: **Haseltine Lake Kempner LLP**
**Lincoln House, 5th Floor**
**300 High Holborn**
**London WC1V 7JH (GB)**

(54)    **APPARATUS, PROGRAM, AND METHOD FOR STORING AND PROCESSING GRAPH DATA**

(57)    Embodiments include an apparatus comprising a memory hardware coupled to a processor hardware; the memory hardware configured to store a data graph encoding information as a plurality of vertices and interconnections between the plurality of vertices, each vertex representing an entity, each interconnection being directed from one vertex to another vertex, representing a relation of the entity represented by the one vertex to the entity represented by the another vertex, and being assigned a label defining the relation; the processor hardware configured to execute processing instructions stored on the memory hardware causing the processor hardware to: receive an identification of a vertex in the data graph as a subject of a query; in respect of each of a plurality of candidate vertices in the data graph, find paths along interconnections between the respective candidate vertex and the subject of the query, the candidate vertices being a group of vertices from among which a closest match to the subject vertex is required by the query; for each interconnection on each of the paths, obtain a word vector representation of the label assigned to the interconnection generated by forward propagating all or part of the label through a neural language model stored on the memory hardware; for each path, computing a distance measure based on a cumulative sum of the obtained word vector representations of the interconnections on the path; identify the path from among the found paths with the shortest computed distance measure, and output, as a response to the query, the candidate vertex in respect of which the identified path was found.

FIGURE 1

**EP 3 499 386 A1**

**Description**

INTRODUCTION

The invention lies in the field of data storage and data processing. In particular, the invention relates to the storage of graph data and the handling of queries relating to the data. Embodiments include apparatus, methods, and programs for outputting medical diagnoses.

The graph model of stored data is considered a faithful reflection of how humans establish and organise our knowledge. A data graph is composed of entities (from the domain of discourse) represented as vertices (or nodes) of the data graph and relations represented as edges (or arcs) of the graph. Entities are labelled to uniquely identify them for both modelling and performance reasons. Edges are labelled as a key channel to define relationships between entities and thus to represent information. Information, or "knowledge" is materialised as the connections/associations among entities.

Graph models are widely used in domains such as the medical domain to indicate the connections among for instance, diseases and symptoms. Edge labels are key contributors to the understanding of semantics conveyed in such graph models. For a small graph with short graph path, the semantic relation among entities can be straightforward: as indicated by the edge labels. For example, a vertex "excessive thirst" may be linked to a vertex "diabetes" by an edge labelled "symptom of". However, longer graph paths may present a challenge to such a semantic explication approach. The combination of edge labels may lead to a semantic explosion or semantic collapse when simply concatenating natural language labels does not reveal any meaningful combination.

Semantics, unlike numeric values, cannot be easily aggregated. Semantic projection or summary of such long graph paths, however, is desirable in many applications.

Embodiments include an apparatus comprising a memory hardware coupled to a processor hardware; the memory hardware configured to store a data graph encoding information as a plurality of vertices and interconnections between the plurality of vertices, each vertex representing an entity, each interconnection being directed from one vertex to another vertex, representing a relation defined by the label of the entity represented by the one vertex to the entity represented by the another vertex, and being assigned a label defining the relation; the processor hardware configured to execute processing instructions stored on the memory hardware causing the processor hardware to perform a process. The process includes: receiving an identification of a vertex in the data graph as a subject of a query; in respect of each of a plurality of candidate vertices in the data graph, finding all paths along interconnections between the respective candidate vertex and the subject of the query; for each interconnection on each of the paths, obtaining a word vector representation of the label assigned to the interconnection generated by forward propagating all or part of the label through a neural language model stored on the memory hardware; for each path, computing a distance measure based on a cumulative sum of the obtained word vector representations of the interconnections on the path; and identifying the path from among the found paths with the shortest computed distance measure, and outputting, as a response to the query, the candidate vertex in respect of which the identified path was found.

Embodiments provide a mechanism for comparing graph vertices by using a semantic aggregation technique based on embedding words as vectors to measure path distance between the vertices. Embodiments implement a similarity based inference for identifying most similar vertices to a subject of a query by aggregating semantics along graph paths.

Embodiments provide an unambiguous approach to the evaluation of distances between vertices in a data graph based on a semantic aggregation of edge labels.

Word vector representations of labels are numeric n-dimensional (where n is typically between 100 and 300) manifestations of how the word or words in the label are used in whatever text data is used to train the neural language model. Therefore, the word vector representations provide an objective quantification of the semantics of a word, which are in a form that can be combined and compared mathematically in order to generate conclusions about the semantics of the word or words represented by said vectors.

Comparing vertices in a graph by measuring a path distance between the two is a computationally efficient way of assessing the similarity between the vertices. Embodiments provide a means to carry out such a comparison in a labelled, directed graph, by quantifying the edge labels as word vectors using a neural language model, so that the path distance can be measured based on semantics of the component edges. This novel form of comparison can explicate similarity in data graphs, for example, in order to spot similarities between patients in a clinical environment so that diagnoses can be compared or new diagnoses generated.

Embodiments may further comprise network input output hardware, the processor hardware further configured to execute processing instructions stored on the memory hardware causing the processor hardware to: acquire, from a data source external to the apparatus via the network input output hardware, text data relating to a knowledge domain to which the information encoded by the data graph belongs; and train the neural language model using the acquired text data, the training comprising modifying weights and biases in the neural language model based on usage of vocabulary in the acquired text data.

Advantageously, such embodiments provide domain-specific word vector representations of the interconnection labels. Therefore, the distance measure between two vertices is based on the semantics of the labels on the path between the two vertices as said semantics are utilised in the relevant domain.

Embodiments may include the obtained word vector representation of the label being obtained as one of: a first vector, being the vector output by the neural language model when the label is forward propagated through the neural language model; and a second vector, being the vector of one of a plurality of cluster centres in the embedded space of word vectors closest to the vector output by the neural language model when the label is input to, and forward propagated through, the neural language model, the plurality of cluster centres being determined by forward propagating through the neural language model each word in a vocabulary to which the labels of the interconnections in the data graph belong, and executing a clustering algorithm on the output word vectors of the vocabulary.

Whether the first vector or second vector is utilised may be down to a user preference. For example, a user of the apparatus may set a preference prior to, or whilst, submitting a query to the apparatus. The first vector provides a direct and individual assessment of each label, and will emphasise small differences in semantics. The second vector utilises clustering, and so words with similar semantics will obtain the same word vector representation. Therefore, small differences in semantics will be understated.

Embodiments may further include, for each interconnection on each of the paths, the assigned label is scanned to detect wording indicating a logical negation, and if the logical negation is detected in the label, in calculating the cumulative sum for the distance measure of the respective path, a third vector orthogonal to the obtained one the first vector and the second vector is used in place of the one of the first vector and the second vector in the cumulative sum.

Advantageously, the third vector represents the logical negation by moving the cumulative sum of the vectors away from where it would have been without the logical negation. Thus, it can be appreciated that two paths identical other than for the logical negation, will result in different cumulative sums.

Embodiments may include each of the found paths being one of: a first arrangement consisting of a monodirectional path defined by one or a series of interconnections from the subject of the query to the respective candidate vertex or vice-versa; and a second arrangement consisting of a bidirectional path defined by two component paths: a first component path being one or a series of interconnections, between the subject of the query and an intermediate vertex, in a first direction being one of two opposing directions: toward the intermediate vertex; and away from the intermediate vertex; a second component path being one or a series of interconnections, between the respective candidate vertex and the intermediate vertex, in a second direction opposite to the first direction.

The two different path arrangements provide constraints on the paths that will be measured and hence constrain the vertices that may be output in response to the query. The logic is that including paths with more than a single joint is not computationally efficient since it increases the number of paths that must be considered, and it can be appreciated that paths with more than a single join can be presumed to indicate too significant a distance along the path for the vertex to be a useful output.

Embodiments may further comprise: if the path is of the first arrangement, computing the distance measure comprises: computing the cumulative sum of the obtained word vector representations of the interconnections on the path; dividing the computed cumulative sum by the number of interconnections on the path; computing a vector norm of the result of the dividing as the distance measure. If the path is of the second arrangement, computing the distance measure comprises: for each of the first component path and the second component path, computing the cumulative sum of the obtained word vector representations of the interconnections on the path; and dividing the computed cumulative sum by the number of interconnections on the path; and computing, as the distance measure, the dot product of the result of the dividing for the first component path and the result of the dividing for the second component path.

Advantageously, such embodiments are equipped to provide a meaningful comparison of paths with different arrangements, by computing path distance measures in a manner which takes into account directionality and the arrangement of interconnections within the path.

Embodiments may be in the medical apparatus domain. In particular, the information encoded by the data graph is in the medical domain and comprises patient records, vertices in the data graph representing patients, diseases, treatments, diagnoses, and symptoms, and interconnections represent symptoms detected in a patient, diagnoses made in response to detected symptoms, diseases identified in the diagnoses, and treatments prescribed in treating an identified disease; the subject of the query is a vertex representing a patient for which a diagnosis is required; the candidate vertices are the other vertices representing patients in the data graph; and the response to the query comprises the candidate vertex in respect of which the identified path was found, along with vertices and interconnections within a maximum number of interconnections of said candidate vertex, including one or more diagnosis vertices.

For instance, in a medical domain, it is possible to have a data graph containing patients together with diseases, treatments, diagnoses, symptoms/signs, etc. When two patients are connected in the graph via a plethora of paths, it will be useful for both computer systems and human interpreters to understand the real semantics of each connecting path and thus to reveal the core semantics of patient connection. Such core semantics can help a clinician to make judgement based on how similar or dissimilar the connected patients are and whether experiences and decisions from one patient can be applied on the connected patients. In particular, the identification of the patient vertex connected to the subject patient vertex by the path having the shortest distance will provide a clinician with a patient whose records may be compared with the subject patient vertex to reveal diagnosis or treatments that may be applied to the patient represented by the subject vertex.

In embodiments, receiving the identification of the vertex in the data graph as a subject of a query may comprise: receiving a portion of graph data; aligning the received portion of graph data with the data graph; and adding the aligned portion of graph data to the data graph including adding one or more interconnections between the aligned portion of graph data and the data graph; the identified vertex in the data graph being an identified vertex from among the aligned portion of graph data.

New data may comprise a subject vertex of a query, along with information about the subject vertex, the information being represented by other vertices and interconnections between the subject vertex and the other vertices. The other vertices may be aligned with vertices already existing in the data graph using reconciliation processing, and thus the portion of new graph data is aligned with the existing graph data and may be added thereto.

According to embodiments, the plurality of vertices may include a plurality of class vertices, with each one of the remaining vertices being classified as belonging to one of the plurality of class vertices, said belonging denoted by an interconnection between the one of the remaining vertices and the respective class vertex, wherein the plurality of candidate vertices comprises all vertices belonging to the same class vertex as the subject of the query.

Class vertices provide a model or ontology to structure the vertices in the data graph. Such class vertices can, in turn, be used to constrain the selection of candidate vertices to ensure that the vertex output in response to the query is comparable to the subject vertex.

Each of the vertices may be an image file (with metadata encoding the graph structure) storing an image of the entity represented by the respective vertex. Relations between image files are revealed by querying the graph, which leverages language processing of defined relationships between image files to identify and quantify similarity between image files. Processing images in this manner is a new and computationally efficient procedure for performing image-matching and image-similarity analysis.

Embodiments of another aspect include a method comprising storing a data graph encoding information as a plurality of vertices and interconnections between the plurality of vertices, each vertex representing an entity, each interconnection being directed from one vertex to another vertex, representing a relation defined by the label of the entity represented by the one vertex to the entity represented by the another vertex, and being assigned a label defining the relation; receiving an identification of a vertex in the data graph as a subject of a query; in respect of each of a plurality of candidate vertices in the data graph, finding paths along interconnections between the respective candidate vertex and the subject of the query; for each interconnection on each of the paths, obtaining a word vector representation of the label assigned to the interconnection generated by forward propagating all or part of the label through a neural language model stored on the memory hardware; for each path, computing a distance measure based on a cumulative sum of the obtained word vector representations of the interconnections on the path; and identifying the path from among the found paths with the shortest computed distance measure, and outputting, as a response to the query, the candidate vertex in respect of which the identified path was found.

Embodiments also include a computer-readable medium storing processing instructions which, when executed by a computing apparatus, cause the computing apparatus to perform a method of an embodiment. Such a computing apparatus may be a server or a network of servers.

Features of embodiments will now be described, purely by way of example, with reference to the accompanying drawings, in which:-

Figure 1 is a flowchart illustrating processes in embodiments;
Figure 2 illustrates a process of vector addition;
Figure 3 illustrates an apparatus of an embodiment; and
Figure 4 illustrates a hardware configuration of an embodiment.

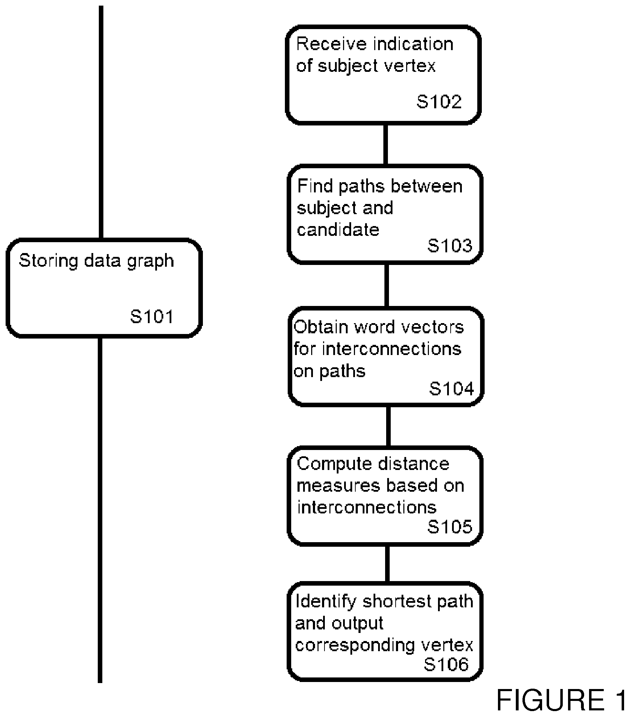

Figure 1 is a flowchart illustrating processes in embodiments. Two parallel process are illustrated. The storing a data graph S101 is performed by memory hardware in cooperation with a control or management program executed by coupled processor hardware. The steps S102 to S106 are a process performed, for example, by processor hardware executing instructions stored on a coupled memory hardware and utilising coupled memory hardware to store data during the execution of each individual step and to make data available for subsequent steps.

Step S101 is a step of storing a data graph. The line through S101 indicates that the storage of the data graph is perpetual with respect to the process of S102 to S106.

The data graph stored by the memory hardware at S101 may also be referred to as graph data, a graph model, or as a graph data model. The data graph encodes information as a plurality of vertices and interconnections between the plurality of vertices, each vertex representing an entity, each interconnection being directed from one vertex to another vertex, representing a relation defined by the label of the entity represented by the one vertex to the entity represented by the another vertex, and being assigned a label defining the relation. Vertices may also be referred to as graph vertices, nodes, or graph nodes. The interconnections may be referred to as edges, links, or connections. The graph data may be encoded as triples, with each triple denoting a labelled, directed interconnection from one identified vertex to another identified vertex. The two vertices identified by a triple may be termed the subject vertex and the object vertex of the triple, with the labelled interconnection being the predicate. In terms of direction, interconnections tend to be directed from subject to object, but this convention may be reversed, as long as directionality is consistent throughout the data graph.

A data graph encodes or represents information or knowledge. The information or knowledge represented by the data graph may be constrained by a specified domain. Domain in this regard is taken to mean an area of endeavour, or an industry to which the data graph is applied. For example, the medical domain, or a sensor network.

Each vertex representing may be taken to mean that there is a correspondence between each vertex and a corresponding entity in the domain represented by the data graph. Similarly, each interconnection representing a relationship may be taken to mean that there is a correspondence between the interconnection and a relationship between two entities in the domain represented by the data graph. The label assigned to the interconnection defines a relation means the label assigns a semantic meaning to the label. Semantic meaning of interconnections in the data graph is not merely a cognitive property subject to human understanding, but, by virtue of step S104, is a quantifiable objective representation of the label, quantified by a word vector representation. The word vector representation represents the use of the word or words in the label in a text corpus used to train a neural language model.

Each of the vertices may be an image file (with metadata encoding the graph structure) storing an image of the entity represented by the respective vertex. Interconnections are labelled with a semantic description of the relationship between two entities in the images. Images of plural entities may be replicated in the data graph, or alternatively, each of the vertices may be a logical or physical pointer to a storage address at which the image file is accessible, and plural vertices may point to the same image file. It is noted that vertices are named after the entity that they represent.

At S102 an indication of a vertex in the data graph as a subject vertex of a query is received by the processor hardware. The indication may be received via a query interface, for example, if the processor hardware is part of a computing apparatus enabling interaction with a user. The interaction with a user may be direct, for example via input/output apparatus such as a mouse, keyboard, microphone, or other such apparatus, or may be indirect and received over a network. The user in this regard may be a human user or may be application software.

At S103, processing is performed by the processing hardware in cooperation with the coupled memory hardware to find all paths between the subject vertex and a group of candidate vertices. The candidate vertices are a group of vertices from among which a closest match to the subject vertex is required by the source of the query. The candidate vertices may be identified via the query interface along with the subject vertex. Alternatively, the candidate vertices may be all vertices belonging to the same classification or categorisation of vertex as the subject vertex, such classification or categorisation indicated interconnections to vertices denoted as class vertices or category vertices in the data graph.

Once the group of candidate vertices are identified, the process, in respect of each of a plurality of candidate vertices in the data graph, finds paths along interconnections between the respective candidate vertex and the subject of the query. The processor hardware may execute a breadth-first search algorithm, a Dijkstra algorithm, or an A* algorithm to perform the path finding. The paths found by the algorithm may be all paths between the subject vertex and the candidate vertex, only the shortest path between the subject vertex and the candidate vertex, or all paths between the subject vertex and the candidate vertex subject to one or more constraints. Such constraints may include, for example, a single joint restriction.

Given two graph vertices, $v_s$ and $v_t$, a path between $v_s$ and $v_t$ is a finite sequence of edges, $\langle v_s, v_0 \rangle, \ldots , \langle v_i, v_j \rangle, \ldots , \langle v_n, v_t \rangle$ that connect a sequence of vertices, $v_s, v_0, \ldots , v_n, v_t$ where the $v_x$ are normally constrained to being distinct.

In many graph analysis problems, $path(v_s, v_t)$ may give more than one result, indicating multiple paths between $v_s$ (wherein $v_s$ is one of the subject vertex and the candidate vertex) and $v_t$ (wherein $v_t$ is the other of the subject vertex and the candidate vertex).

Among them, the shortest path indicates one with the short path length. When searching for path(s) between two vertices, established algorithms can be used. For instance, naïve breadth-first search can be applied to iteratively check all the neighbours of a vertex. For shortest path, Dijkstra algorithm or A* algorithm are efficient solutions.

In embodiments, graphs are directed, that is to say, an interconnection with label "X" from vertex A to vertex B does not equal an interconnection also with label "X" from vertex B to vertex A. Graph path finding in embodiments may observe "single joint" restriction when finding and numerating the paths. The single joint restriction is defined as follows:

A path $\langle v_s, v_0 \rangle, \ldots , \langle v_i, v_j \rangle, \ldots , \langle v_n, v_t \rangle$ satisfies one of

the two conditions:

1. There is a direct path from $\overrightarrow{\langle v_s, v_0 \rangle}, ..., \overrightarrow{\langle v_i, v_j \rangle}, ..., \overrightarrow{\langle v_n, v_t \rangle}$ from $v_s$ to $v_t$; or

2. There is one and only one interim vertex $v_j$ such that $\overrightarrow{\langle v_s, v_0 \rangle}, ..., \overrightarrow{\langle v_i, v_j \rangle}, ..., \overleftarrow{\langle v_n, v_t \rangle}$ from $v_s$ to $v_t$.

In other words, a permissible path found in S103, is either a directed path from $v_s$ to $v_t$ or two directed paths from $v_s$ to $v_j$ and from $v_t$ to $v_j$ connecting at joint vertex $v_j$, where there is no other joint vertices on the path. In practice, this constraint can be realised by first treating the graph as undirected and detecting all the paths among given graph vertices and then adding the directional information to the paths and filtering the paths based on edge directions and number of joint vertices (i.e. interim vertices fulfilling the restrictions of $v_j$ in point 2 above).

The edge direction information will be preserved in the found paths, and the found paths stored for processing in steps S104 to S105.

At steps S104 to S105, each of the paths found for each of the candidate vertices is measured. As a first step, at S104, word vector representations of the semantic labels assigned to the interconnections composing each path are obtained. At step S105, the word vector representations for each path are converted into a single scalar quantity representing distance from one end of the path to the other.

At S104, for each interconnection on each of the paths, the processor hardware obtains a word vector representation of the label assigned to the interconnection generated by forward propagating all or part of the label through a neural language model stored on the memory hardware. The word vector representations may have been generated prior to the initiation of this particular process (i.e. execution of steps S102 to S106) as a pre-processing step for some or all interconnections, and stored on the memory hardware as part of or in association with the data graph. For example, the word vector representation of an interconnection label may be generated each time a new label is added to the data graph, and stored, so that new additions of the same label are associated with the stored word vector representation. Alternatively, the word vector representations may be generated on-the-fly following the execution of step S103. That is say, the obtaining at S104 may comprise generating the word vector representations, or may comprise recalling from storage previously-generated word vector representations.

A neural language model is a language model based on a neural network, and leverages the ability of the neural network to learn statistical characteristics.

Typically, neural language models output predictions of a next word in a sequence given one or more preceding words. In order to perform this function, words are projected into an embedded space, in other words, words are given a word vector representation. In the embodiments, it is the word vector representations that are utilised from the neural language model. The neural language model is trained with a text corpus. The text corpus comprises natural language, and statistical characteristics describing the usage of words in terms of proximity to other words are learned by the neural language model and exposed in the word vector representation (which may be referred to as distributed representation) of the word.

Word vectors effectively describe how a word or a term/phrase is used within the context of a text corpus. By projecting all the words/terms on to a vector space, with the assumption that semantically and syntactically similar words normally will appear in the same linguistic context, co-occurring with similar words, they will be projected to positions having close spatial proximity. Therefore, characteristics of the high-dimensional word vector space reflect characteristics of word semantics.

Each edge on the found graph paths is labelled with a word/term. By forward propagating all or part of the label through a neural language model stored on the memory hardware, a word vector representation of the label is obtained. The use of all or part of the label is determined by pre-processing before forward propagating the label through the neural language model. Edge labels normally only consist of one or two words, and indeed, the data graph may be constrained to limit edge labels to comprising one or two words.

The edge labels are pre-processed to acquire the word or term that will be input to the neural language model. In exemplary pre-processing, edge labels are processed to remove common stop words and perform plural and tense folding. For example, one or more of the following simplification rules may be applied:

a) if both verb and adverb present, remove adverb, generate word vector representation of verb;
b) if both verb "to be", in any form, and adjective present, remove "to be", generate word vector representation of adjective;
c) if more than one verb present, treat the edge as a "multiple edge", that is, an edge with multiple versions, one for each of the juxtaposed verbs, obtain a word vector representation of each version (and in path finding S103 treat the remainder of the path with each version of the multiple edge as a separate path, thereby increasing the original graph path count between subject vertex and respective candidate vertex);
d) similarly to c), if more than one adjective present, treat the edge as a multiple edge, with one version for each of the juxtaposed adjectives, obtain a word vector representation of each version (and in path finding S103 treat the remainder of the path with each version of the multiple edge as a separate path, thereby increasing the original graph path count between subject vertex and respective candidate vertex);

e) optionally, clustering is executed on the word vector representations of the vocabulary of the text corpus used to train the neural language model, so each word vector representation generated in a) to d) belongs to a cluster. In which case, the word vector representation is modified to being the cluster centre of the cluster to which the word vector generated in a) to d) belongs;

f) Handling logic negation of edge label: word vector representation of the label will be adjusted based on edge labels. For instance an edge labelled "not_effective" will be adjusted as orthogonal vector of "effective" to reflect the logic negation.

For instance, if a graph edge is <anti-inflammatory, treats, gout>, the vector embedding the edge (i.e. the word vector representation of all or part of the label) will be the word vector representation of "treats" or "treat". Also, if a graph edge is <anti-inflammatory, effectively-treats, gout>, the vector embedding the edge will be the word vector representation of "treats" or "treat".

The word vector representation of all or part of the label assigned to an interconnection/edge on the graph may be referred to as an edge vector or an interconnection vector, wherein "edge" and "interconnection" are interchangeable.

At S105, for each path found in S103, a distance measure is computed based on the word vector representations of the labels assigned to the interconnections on the respective paths obtained in S104. At S105, the processor hardware computes a distance measure for each path based on a cumulative sum of the obtained word vector representations of the interconnections on the path. The distance measure is a quantification of the path length.

The computed distance measure is based on a cumulative sum of the obtained word vector representations of the interconnections on the path.

The cumulative sum is the vector addition of the word vector representations of labels assigned to interconnections on the path.

Exemplary techniques for calculating the cumulative sum and path distance measures are as follows:

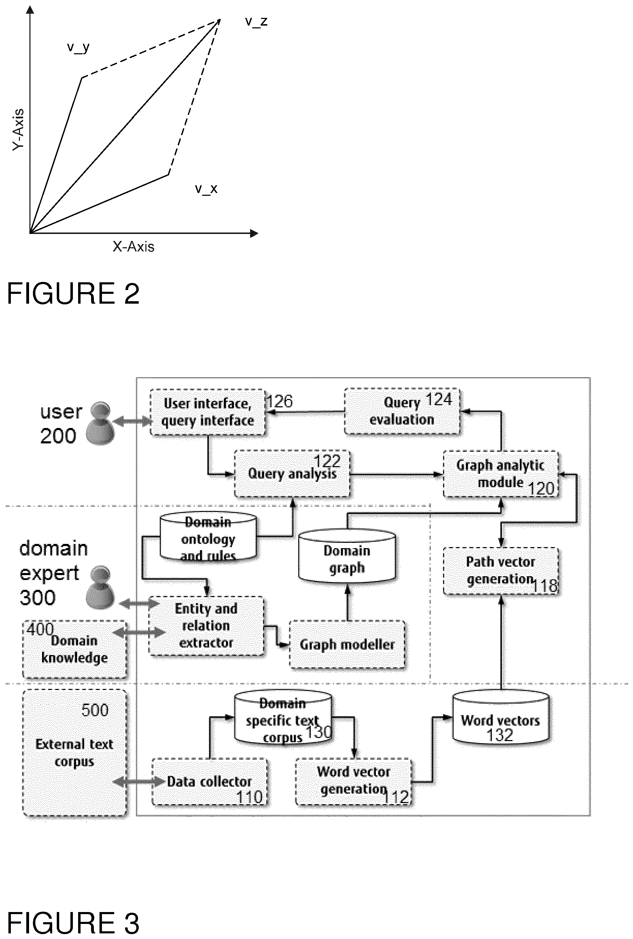

1. Adding edge vectors:
The word vector representation of the labels assigned to the interconnections on the path have been generated or recalled from storage in step S104. The obtained word vector representations are summed by vector addition of the word vector representations of labels assigned to each of the edges along the path. This is illustrated in Figure 2, for the simplified example of a 2-dimensional space. In Figure 2, v_x and v_y are the word vector representations of labels assigned to individual edges on the path, and v_z is the result of vector addition of the two word vectors. The technique leverages the assumption that word embedding vectors may be subject to addition and subtraction and the results of such vector operations are semantically sound.

The cumulative sum obtained by the vector addition of the word vector representations of labels assigned to edges on the path may be moderated by a normalisation component to obtain a path vector, a vector representation of the entire path. For example, the path vector may be:

$$v_{(v_s,v_t)} = \frac{\sum_{(v_i,v_j)\in(v_s,v_t)} v_{vi,vj}}{\left|(v_s,v_0),\dots,(v_i,v_j),\dots,(v_n,v_t)\right|}$$

This is average of cumulative sum of all the edge vectors, where the denominator gives the number of edges (or the length of path) to normalise the sum. In other words, the path vector is the cumulative sum of the edge vectors divided by the number of edges on the path. In the above equation, $v_s$ is the subject vertex, $v_t$ is the candidate vertex, and $v_0, \dots, v_n$ are vertices joined by interconnections on the path from $v_s$ to $v_t$. The brackets indicate an edge between the two vertices denoted between the brackets. The v with a subscript indicates a vector between the vertices indicated in the subscript.

2. When a path is a single joint path (as defined above), the technique for calculating the cumulative sum is different to that specified in 1, to take into account the directionality of the edges on the path. A path vector is defined as follows: let path ($v_s, \dots, v_{joint}, \dots, v_t$), where $v_s$ is the starting vertex, $v_t$ is the ending vertex, and $v_{joint}$ the vertex where paths of opposite direction meet:

    a. Summarise and generate a first component path vector of $\overrightarrow{V} = (v_s, \dots, v_{joint})$ using the technique specified above in 1.
    b. Summarise and generate a second component path vector of $\overleftarrow{V} = (v_{joint}, \dots, v_t)$ using the technique specified above in 1.
    c. Optionally, the overall path vector can be calculated as the cross product of the two joining edge vectors, that is, the two vectors obtained in steps a and b.

Once the path vectors are obtained, a distance measure is computed and assigned to each path. For example, the following technique may be used for computing distance measure:

1. For directed path (path where no edge direction is reversed), the similarity is computed as the cumulative sum of all the edge vectors from starting vertex to the end vertex. Optionally, it is the cumulative sum moderated by the number of edges that is used. The cumulative sum emulates the joined effect of the different edge vectors on the starting vertex. The overall similarity, that is, the path distance measure, is then computed as the norm of the path vector calculated in step 1 above, $\|v\|$.

2. For a single joint path, for which two component path vectors are generated in 2a and 2b above, the similarity, that is, the distance measure of the path between starting and ending vertex can be computed as the dot-product of the two component path vectors at the joint vertex. Effectively, this equals computing the cosine value of the angle made by the two component path vectors.

$$\vec{v} \cdot \vec{\bar{v}} = \cos(\vec{v}, \vec{\bar{v}})$$

At step S106, a smallest path distance measure from among those computed in S105 is identified by the processor hardware. The candidate vertex in respect of which the path having the smallest path distance measure is output in response to the query. The destination of the output is implementation dependent, but may be, for example, in the case of the query being received via a query interface (which may be an element of a user interface), the query interface, or, in the case of the query being received from an application, an interface to the application.

The candidate vertex may be output along with graph data within one, two, or n, interconnections of the candidate vertex. The graph data may be presented as graph data or may be presented, for example, as text statements, or presented in some other format. In examples in which the vertices in the graph data are classified, the query may specify a classification of vertices for inclusion in the query response, and the candidate vertex may be output along with vertices belonging to the specified classification to which the candidate vertex is connected via an interconnection. For example, in the medical domain, vertices of the class "diagnosis" may be requested and output, so that the apparatus operates as a diagnosis machine.

Figure 3 illustrates a system architecture of an apparatus of an embodiment.

The system of Figure 3 includes the apparatus 100 of an embodiment, an apparatus user 200, a domain expert 300, domain knowledge 400, and an external text corpus 500.

The apparatus 100 comprises processor hardware, memory hardware, and input/output hardware. Within the apparatus 100, the components illustrated as cylindrical are stored data, and hence correspond to areas within the memory hardware. The components illustrated within dashed boxes are processes performed by the processor hardware, and hence correspond to processing instructions stored by the memory hardware and executed by the processor hardware. Arrows from a process to stored data indicate the generation or modification of the stored data by the process. Arrows from stored data to a process indicate the reading out and utilisation of the stored data by the process. Arrows between a process within the apparatus 100 and an entity outside of the apparatus indicate the use of input/output hardware by the process. The arrows are intended to illustrate the principle data exchanges, and are not exclusive.

The apparatus may simply access an already existing data graph, stored on memory hardware accessible to the apparatus 100. In the apparatus of Figure 3, components entity and relation extractor 114, and graph modeller 116, are optional processes for the construction of a data graph 134. The domain ontology and rules 136 are used by the graph modeller 116 in constructing the domain graph 134. The domain ontology and rules are domain specific information representing the domain being modelled.

The domain entity and relation extractor 114 and graph modeller 116, are processes to facilitate the construction of graph representation of domain knowledge 400 by a domain expert 300. In practice, many methods can be applied. Human intervention or interaction is facilitated via the domain expert 300 interacting with the entity and relation extractor 114. The human domain experts 300 are responsible for either manually defining the knowledge representation model or supervising automated ontology learning algorithms. The graph construction may be based on given domain ontologies which can specify key concepts and relationships of the domain of discourse. The outcome of the graph modeller 116 is a domain graph with vertices corresponding to entities (concepts and instances) in the domain of discourse (bearing labels to the named concepts and instances) and edges corresponding to relationships among the vertices. Edges are attributed meaning that they bear labels as names and potentially other metadata.

The domain graph 134 may be referred as the data graph, and may be stored on one or a network of servers, the servers forming part of the memory hardware of the apparatus 100. Optionally, an edge oriented storage solution enhanced with path completeness may be utilised for storing the domain graph 134. Edge oriented storage ensure complete information about one edge is stored on a single server. Path completeness stores consecutive edges (e.g. ⟨s, z⟩, ⟨z, x⟩, ⟨x, t⟩, when the co-occurrence of vertices s, t are significantly higher than a predefined threshold), on the same server. Some dynamic locality approaches can also be leveraged to determine physical division of the domain graph 134 among plural servers.

The data collector is to monitor and interact with external data sources to collect raw text data based on given conditions (eg. one or more keywords input by a user 200). The data collector 110 acquires text data relevant to the domain represented by the data graph from an external text corpus 500, using the input/output hardware of the apparatus 100. For example, via a network interface card. The data collector stores the domain specific text corpus 130 on the memory hardware of the apparatus. The word vector generation process 112 trains the neural language model, stored on the memory hardware, using the domain specific text corpus 130. The outcome of the word vector generation process 112 is real-valued vectors for each of a vocabulary of words or fix terms. The domain specific text corpus is the internal storage for collected data. A storage method used for the domain specific text corpus may be, for example, a MongoDB document storage solution.

In order for the embedding to be representative, the text data acquired by the data collector 110 may be constrained to being above a minimum size, specified by data storage space, words, pages, or characters. If the graph is domain specific, the text data may be constrained to being centred on the same and related domains. For example, the data collector may have, for each of one or more domains, a specified one or more data sources from which text data is acquired. Such one or more data sources may be predetermined, or may be set by a domain expert 300.

As a specific example, the data collection of medical domain may be carried out as follows as an automated process by the data collector 110:

> 1. For a specific domain set in advance by a user 200, e.g. medicine, or a specific subdomain, e.g. mental health, obtain an indication of the domain or subdomain
>
> 2. Enrich the core keyword with related ones based on, for instance, Wikipedia Collection Page for relevant domain keywords, or general Wikipedia Page with high frequency words (after removing stop words using generic stop word lists/dictionaries).
>
> 3. Use the enriched keyword set to query data from either a dedicated, peer-reviewed data source (e.g. for medical domain from PubMed using the dedicated API, or for a particular technology domain use a technology-specific journal or news site, using general internet search engine or site-specific APIs).
>
> 4. Extract text only data and save such data as the domain specific text corpus 130 for further analysis.

It may be necessary to periodically update / pull data from the selected data sources to update the domain specific text corpus 130 with up-to-date information. Such a periodical update may be carried out by automated web crawling software with pre-set time windows. In practice, the size of text data with respect to a particular domain (not very narrow ones) may be constrained to being more than 1 gigabyte, where duplication is permitted.

The language model is a neural language model and is stored along with the domain specific text corpus 130 and used by the word vector generation process 112 to generate word vectors 132 for the vocabulary of words appearing in the domain specific text corpus 130. The word vectors are stored in a memory area 132 and used as the word vector representation of an interconnection assigned a label with the same word. The word vector memory area 132 is internal (to the apparatus) storage for word vectors. If plural servers are required for this purpose, technology such as a distributed hash table may be used.

The apparatus 100 may obtain a pre-trained neural language model, or may be configured to train the neural language model using the domain specific text corpus 130. Based on the text data in the corpus, the neural language model is trained and word vectors obtained as the by-product of neural text embedding. For example, the training and obtaining word vectors may be performed as follows:

> 1. Carry out natural language processing (NLP) preprocessing on articles from the text store. NLP preprocessing may include one or more from among: tense and plural folding, stemming, and generic as well as domain-specific named entity recognition (NER). For NER, if a domain specific dictionary is accessible to the apparatus 100, it may be exploited to improve overall accuracy. In the absence of access to such a dictionary, generic NER rules or models may be applied. The applying NER is to avoid biases caused by distorted co-occurrence of fixed names and terms.
>
> 2. Train the neural language model. For example, the training may be performed by open source software. Parameter tuning may be necessary to achieve the best results. When training using word based software, e.g. Word2Vec, fixed word combination can be concatenated using for instance hyphens.
>
> 3. Obtain word/term vectors. Depending on the vector size given to the training software, word/term vector size can vary from 100-300, with real numbers, indicating a point in the high dimensional space, that is the embedded space to which the words terms are projected by the neural language models. Word vectors are generated and stored, the word vectors may be generated and stored for all words in the vocabulary of the text data.

The word/term vectors are by-products of using an algorithm to train the weights and biases of the neural language model.

Optionally, the neural language model may be retrained based on previous weights and biases (instead of random initialisation), either periodically based on a pre-defined time threshold or event-triggered when updates to the external text corpus 500 (assuming the apparatus 100 is configured to monitor the external text corpus for updates) or to the local domain specific text corpus 130 (assuming the data collector 110 crawls the external text corpus 500 and downloads updates in an automated manner) the overall change $\Delta$ to be over a predefined threshold. $\Delta$ may be computed as one of the following:

1. The number of newly crawled documents is over a predefined percentage of the document/text store since last training;
2. The size ratio between documents newly-added since last training and old documents is over a predefined threshold.

Since re-training procedures utilise a grounded initial value the overall number of iterations for the training process to converge is reduced relative to starting with random or default initial values.

Word vectors effectively describe how a word or a term/noun phrase is used within the context of a text corpus. By projecting all the words/terms from the vocabulary of the text corpus onto a vector space, with the assumption that semantically and syntactically similar words normally will appear in the same linguistic context, co-occurring with similar words, they will be projected upon to positions having close spatial proximity. Therefore, characteristics of the high-dimensional word vector space represent characteristics of word semantics.

Optionally, in order to reduce errors introduced through inaccurate vector "positions", the word/term vectors may be subjected to an unsupervised clustering process to identify "semantically" close words/terms. Centres of different clusters will be computed and leveraged in further processing.

The clustering may be computed with a clustering algorithm such as K-means, if some prior knowledge of the domain and the text data used to train the neural language model is assumed. Initially K categories are selected and all the word/term vectors are clustered based on the same distance and classification criteria.

If no prior domain knowledge is assumed, an expectation-maximisation algorithm may be applied to optimise a cost function until the process converges.

For both categories of algorithms, existing open-source software libraries and tools may be employed. For example, the K-means clustering algorithm could be the MapReduce of the Mahout package. The expectation-maximisation algorithm may employ Gaussian Mixture, M step, or E step procedures. For example, the k-MLE algorithm.

Once clustered, the cluster centre may be computed as the element-wise mean of all the vectors in the cluster. These cluster centres are considered the representative values of clusters to be used in further computation. That is, a word vector representation of a word that is within the cluster is taken to be the cluster centre in the processing of step S104, obtaining word vectors for interconnections on paths.

The apparatus 100 is equipped with hardware and software for interacting with end users 200. In particular, the apparatus includes:

1. User / query interface 126: This component interacts with end users. For example, the user / query interface 126 executes step S102 and S106 of Figure 1. The user/query interface receives a query from a user, for instance, a description of a patient case. This raw input is then translated into internal representation, that is, the input is aligned with the data graph 134. After evaluating the queries, the user/query interface 126 outputs a response to the user 200. For instance, if it is a medical application, by receiving a patient case, the apparatus will present a list of one or more similar patients that are considered relevant to the input case, and present a diagnosis or suggested action to be taken on the input case, based on highly relevant cases.

2. Query analysis process 122: the query analysis component 122 is to convert input data into a subgraph (centred on a core vertex, e.g. patient vertex) that is aligned with the domain graph 134, using the set of domain ontologies and extraction patterns 136 used to obtain the domain graph 134. The output of the query analysis process 122 is a subgraph that is added to the existing domain graph 134 including at least one interconnection between the subgraph and the existing domain graph 134.

3. Graph analytic process 120: the graph analytic process 120 is to execute steps S103 to S105 of the method of Figure 1, in collaboration with the path vector generation process 118. For example, the graph analytic process 120 is to analyse the domain graph to carry out firstly path finding that iterates all the alternative paths between graph vertices that are of the same type as the core vertex of the input subgraph (in many cases, this can be ego-centric network); secondly, to calculate path vectors by adding word vectors for labels assigned to interconnections on the paths; and thirdly, compute the path distance measure for each path.

4. Path vector generation process 118: the path vector generation process 118 is to iterate all alternative paths between the centre vertex of the query (i.e. the subject vertex of the query) and all candidate vertices, which may be all other vertices of the same type. The found paths may be cached in a temporary storage (physical or in memory) for further analysis by the graph analytic process 120.

5. Query evaluation process 124: the query evaluation process 124 takes the path distance measures computed by the graph analytic module 120 and produces a list of one or more vertices representing entities from the domain graph 134 semantically similar to the subject of the query. The query evaluation process 124 then sorts and filters the candidate cases to highlight the top ranking ones, that is to say, to identify one or a predetermined number of vertices connected to the subject vertex by paths having the shortest or smallest path distance measure. Furthermore, the query evaluation process may also aggregate metadata and/or connected vertices from the identified one or more vertices to generate advised decisions, such as diagnoses in the medical domain. For instance, the query evaluation process 124 may summarise or aggregate treatments and diagnosis of previous similar cases to produce a suggestion on the current case. Aggregation may be done, for example, with majority-vote or other statistical methods.

Embodiments may be applied in graph based analysis and graph based semantic explication that can help in query answering processes. In many technical applications, domain knowledge and domain entities are represented as graph and graph vertices (as well as relations among graph vertices). For instance, a medical domain may be represented as a domain graph representing, as vertices: persons (being patients, medical professionals, and account payers such insurers and local authorities), medications (being drugs and treatments), equipment (being diagnostic and therapeutic machines), organisations (being hospitals and clinical centres), and symptoms and signs; and representing relations between those entities as labelled interconnections. All such entities and interconnections compose a domain graph. The domain graph provides a knowledge base based on which inference and decisions can be made.

In order to reach a conclusion of a current case, operatives in a technical domain may refer retrospectively to previous cases and their corresponding decisions. The operatives may wish to draw conclusions from cases that are semantically similar to a current case. For instance, when receiving, as a query, a patient case (being added to the graph as a graph entity with properties referring to other graph entities, effectively composing an ego-net), embodiments:

    1. Scan the entire domain graph to select candidate vertices belonging to the same class as the subject of the query: in this case all the patient vertices.
    2. Find paths among the subject vertex and the candidate vertices: in this case, the path between the subject patient vertex and all other candidate patient vertices.
    3. Summarise the alternative paths among vertices to identify candidate vertices having the shortest computed path length to the subject vertex: in this case, patient vertices that are considered more semantically relevant to the subject patient are identified to a user, along with connected vertices representing, for example, previous diagnoses.

Once a similar patient or patients are identified and output, decisions made on such similar patients can then be reviewed by the user and referenced to contribute to the decision of the current patient. This is normally referred to as case-based inferences or case-based diagnosis and is in line with how human experts make decisions against unknown cases.

Similarly, the same method can be applied to reveal hidden relations among, for instance sensors within a network of sensors modelled as a domain graph. Fault diagnosis can be prohibitively complex in systems of sensors, for example medical sensors, with plural types of sensors and plural types of relations between the sensors. It is useful in such implementation scenarios to summarise a composite path between entities that are not direct neighbours to understand their relationship for further inference. In practice, with a domain knowledge graph, users can select two entities (e.g. two sensors), the system should then establish the paths among them and semantically summarise the path(s) to provide the answers. Sensors and, for example, data servers, data processors, and other hardware, are modelled as vertices in a domain graph. The data communication between entities is modelled as interconnections in the domain graph. Semantic labels assigned to the interconnections represent the data being communicated.

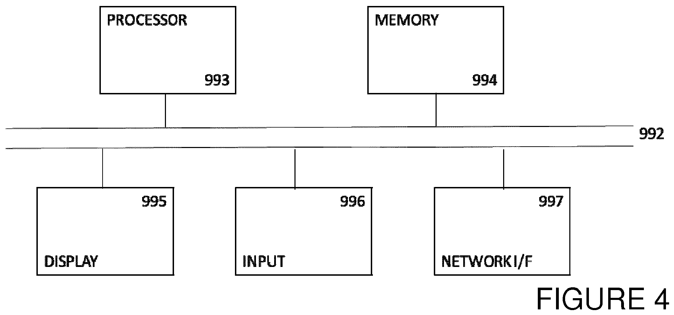

FIGURE 4 is a block diagram of a computing device, such as a server, which embodies the present invention, and which may be used to implement a method of an embodiment of Figure 1. The computing device comprises a processor 993, and memory, 994. Optionally, the computing device also includes a network interface 997 for communication with other computing devices, for example with other computing devices of invention embodiments.

For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different processes illustrated in the apparatus diagram of Figure 3. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device and may also display a cursor and dialog boxes and screens enabling interaction between a user and the programs and data stored on the computing device. The input mechanisms 996 may enable a user to input data and instructions to the computing device.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

The data collector 110 of Figures 3 may be a processor 993 (or plurality thereof) executing processing instructions (a program) stored on a memory 994 and acquiring data from an external data source via a network I/F 997. In particular, the processor 993 executes processing instructions to receive, via the network I/F, text data from an external text corpus and store the data in the domain specific text corpus 130, as illustrated in Figure 3. Furthermore, the processor 993 may execute processing instructions to store the domain specific text corpus 130 on a connected storage unit.

The word vector generation process 112 of Figure 3 may be a processor 993 (or plurality thereof) executing processing instructions (a program) stored on a memory 994. In particular, the processor 993 executes processing instructions to read the vocabulary of the domain specific text corpus 130 and input words from the vocabulary to the trained neural language model to generate word vectors which are stored and then read from memory in step S104 of the method of Figure 1. Furthermore, the processor 993 may execute processing instructions to store the word vectors on a local word vector storage area 132.

The user/query interface 126 of Figure 3 may be a processor 993 (or plurality thereof) executing processing instructions (a program) stored on a memory 994 and exchanging data via a network I/F 997. In particular, the processor 993 executes processing instructions to receive, via the network I/F, query data from a user 200 and to output a response to the query, as in steps S102 and S106 of the method of Figure 1. Furthermore, the processor 993 may execute processing instructions to store the query data on a connected storage unit for addition to the domain graph 134 by the query analysis process 122.

The query analysis process 122 of Figures 3 may be a processor 993 (or plurality thereof) executing processing instructions (a program) stored on a memory 994. In particular, the processor 993 executes processing instructions to read the received query data from storage and align the query data with the domain graph 134. Furthermore, the processor 993 may execute processing instructions to store the query data as part of the domain graph 134 and/or to transmit all or part of the query data t the graph analytic process 120 for further processing.

The graph analytic process 122 of Figures 3 may be a processor 993 (or plurality thereof) executing processing instructions (a program) stored on a memory 994. In particular, the processor 993 executes processing instructions to receive the query data, in particular the subject vertex of the query, and to execute path finding, as in step S103 of Figure 1. Furthermore, the processor 993 may execute processing instructions to store the found paths on a connected storage unit and/or to transmit the found paths to the path vector generation process 118 for further processing.

The path vector generation process 118 of Figure 3 may be a processor 993 (or plurality thereof) executing processing instructions (a program) stored on a memory 994. In particular, the processor 993 executes processing instructions to read the found paths from storage, and obtain word vectors for interconnections on the path from word vector memory area 132, and then to compute the path distance measure for each path, as in steps S104 to S105 of the method of Figure 1. Furthermore, the processor 993 may execute processing instructions to store the computed path lengths on a connected storage unit.

The query evaluation process 124 of Figure 3 may be a processor 993 (or plurality thereof) executing processing instructions (a program) stored on a memory 994. In particular, the processor 993 executes processing instructions to identify the shortest path distance measure from among those calculated by the path vector generation process 118 and to obtain, from the domain graph, the corresponding candidate vertex along with related data. Furthermore, the processor 993 may execute processing instructions to output the data via the query/user interface 126.

Methods embodying the present invention may be carried out on a computing device such as that illustrated in Figure 4. Such a computing device need not have every component illustrated in Figure 4, and may be composed of a subset of those components. A method embodying the present invention may be carried out by a single computing device in communication with one or more data storage servers via a network. The computing device may be a server itself storing the data graph, as in step S101 of Figure 1.

A method embodying the present invention may be carried out by a plurality of computing devices operating in cooperation with one another. One or more of the plurality of computing devices may be a data storage server storing at least a portion of the data graph, as in step S101 of Figure 1.

**Claims**

1. An apparatus comprising:

   a memory hardware; and
   a processor hardware, the memory hardware being coupled to the processor hardware;
   the memory hardware configured to store a data graph encoding information as a plurality of vertices and interconnections between the plurality of vertices, each vertex representing an entity, each interconnection being directed from one vertex to another vertex, representing a relation of the entity represented by the one vertex to the entity represented by the another vertex, and being assigned a label defining the relation;
   the processor hardware configured to execute processing instructions stored on the memory hardware causing the processor hardware to:

      receive an identification of a vertex in the data graph as a subject of a query;
      in respect of each of a plurality of candidate vertices in the data graph, find paths along interconnections between the respective candidate vertex and the subject of the query, the candidate vertices being a group of vertices from among which a closest match to the subject vertex is required by the query;

      for each interconnection on each of the paths, obtain a word vector representation of the label assigned to the interconnection generated by forward propagating all or part of the label through a neural language model stored on the memory hardware;
      for each path, compute a distance measure based on a cumulative sum of the obtained word vector representations of the interconnections on the path;
      identify the path from among the found paths with the shortest computed distance measure, and output, as a response to the query, the candidate vertex in respect of which the identified path was found.

2. The apparatus according to claim 1, wherein each of the vertices is an image file storing an image of the entity represented by the respective vertex.

3. The apparatus according to claim 1 or 2, further comprising:

   network input output hardware;
   the processor hardware further configured to execute processing instructions stored on the memory hardware causing the processor hardware to:

      acquire, from a data source external to the apparatus via the network input output hardware, text data relating to a knowledge domain to which the information encoded by the data graph belongs; and
      train the neural language model using the acquired text data, the training comprising modifying weights and biases in the neural language model based on usage of vocabulary in the acquired text data.

4. The apparatus according to any of claims 1 to 3, wherein, the obtained word vector representation of the label is obtained as one of:

      a first vector, being the vector output by the neural language model when the label is forward propagated through the neural language model; and
      a second vector, being the vector of one of a plurality of cluster centres in the embedded space of word vectors closest to the vector output by the neural language model when the label is input to, and forward propagated through, the neural language model, the plurality of cluster centres being determined by forward propagating through the neural language model each word in a vocabulary to which the labels of the interconnections in the data graph belong, and executing a clustering algorithm on the output word vectors of the vocabulary.

5. The apparatus according to claim 4, wherein for each interconnection on each of the paths, the assigned label is scanned to detect wording indicating a logical negation, and if the logical negation is detected in the label, in calculating the cumulative sum for the distance measure of the respective path, a third vector orthogonal to the obtained one the first vector and the second vector is used in place of the one of the first vector and the second vector in the cumulative sum.

6. The apparatus according to any of claims 1 to 5, wherein
each of the found paths is one of:

a first arrangement consisting of a monodirectional path defined by one or a series of interconnections from the subject of the query to the respective candidate vertex or vice-versa; and
a second arrangement consisting of a bidirectional path defined by two component paths:

a first component path being one or a series of interconnections, between the subject of the query and an intermediate vertex, in a first direction being one of two opposing directions:

toward the intermediate vertex; and
away from the intermediate vertex;

a second component path being one or a series of interconnections, between the respective candidate vertex and the intermediate vertex, in a second direction opposite to the first direction.

7. The apparatus according to claim 6, wherein, if the path is of the first arrangement, computing the distance measure comprises:

computing the cumulative sum of the obtained word vector representations of the interconnections on the path;
dividing the computed cumulative sum by the number of interconnections on the path;
computing a vector norm of the result of the dividing as the distance measure; and

if the path is of the second arrangement, computing the distance measure comprises:

for each of the first component path and the second component path:

computing the cumulative sum of the obtained word vector representations of the interconnections on the path; and
dividing the computed cumulative sum by the number of interconnections on the path; and

computing, as the distance measure, the dot product of the result of the dividing for the first component path and the result of the dividing for the second component path.

8. The apparatus according to any of claims 1 to 7, wherein
the information encoded by the data graph is in the medical domain and comprises patient records, vertices in the data graph representing patients, diseases, treatments, diagnoses, and symptoms, and interconnections represent symptoms detected in a patient, diagnoses made in response to detected symptoms, diseases identified in the diagnoses, and treatments prescribed in treating an identified disease;
the subject of the query is a vertex representing a patient for which a diagnosis is required;
the candidate vertices are the other vertices representing patients in the data graph; and
the response to the query comprises the candidate vertex in respect of which the identified path was found, along with vertices and interconnections within a maximum number of interconnections of said candidate vertex, including one or more diagnosis vertices.

9. The apparatus according to any of claims 1 to 8, wherein
receiving the identification of the vertex in the data graph as a subject of a query comprises:

receiving a portion of graph data;
aligning the received portion of graph data with the data graph; and
adding the aligned portion of graph data to the data graph including adding one or more interconnections between the aligned portion of graph data and the data graph;
the identified vertex in the data graph being an identified vertex from among the aligned portion of graph data.

10. The apparatus according to any of claims 1 to 9, wherein
the plurality of vertices includes a plurality of class vertices, with each one of the remaining vertices being classified as belonging to one of the plurality of class vertices, said belonging denoted by an interconnection between the one of the remaining vertices and the respective class vertex, wherein
the plurality of candidate vertices comprises all vertices belonging to the same class vertex as the subject of the query.

11. A method comprising:

storing a data graph encoding information as a plurality of vertices and interconnections between the plurality of vertices, each vertex representing an entity, each interconnection being directed from one vertex to another vertex, representing a relation of the entity represented by the one vertex to the entity represented by the another vertex, and being assigned a label defining the relation;

receiving an identification of a vertex in the data graph as a subject of a query;

in respect of each of a plurality of candidate vertices in the data graph, finding paths along interconnections between the respective candidate vertex and the subject of the query, the candidate vertices being a group of vertices from among which a closest match to the subject vertex is required by the query;

for each interconnection on each of the paths, obtaining a word vector representation of the label assigned to the interconnection generated by forward propagating all or part of the label through a neural language model stored on the memory hardware;

for each path, computing a distance measure based on a cumulative sum of the obtained word vector representations of the interconnections on the path; and

identifying the path from among the found paths with the shortest computed distance measure, and outputting, as a response to the query, the candidate vertex in respect of which the identified path was found.

12. A computer program which, when executed by a computing apparatus, causes the computing apparatus to execute the method of claim 11.

13. A non-transitory computer-readable medium storing the computing program according to claim 12.

Storing data graph

S101

Receive indication
of subject vertex

S102

Find paths between
subject and
candidate

S103

Obtain word vectors
for interconnections
on paths

S104

Compute distance
measures based on
interconnections

S105

Identify shortest path
and output
corresponding vertex

S106

FIGURE 1

FIGURE 2

FIGURE 3

PROCESSOR

993

MEMORY

994

992

995

DISPLAY

996

INPUT

997

NETWORK I/F

FIGURE 4

Europäisches
Patentamt

European
Patent Office

Office européen
des brevets

**EUROPEAN SEARCH REPORT**

Application Number

EP 17 20 7148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/347629 A1 (PAJOR THOMAS [US] ET AL) 3 December 2015 (2015-12-03)<br>* figures 1, 9 *<br>* paragraph [0021] - paragraph [0080] *<br>----- | 1-13 | INV.<br>G06F17/30<br>G06F19/00<br>G16H50/20 |

TECHNICAL FIELDS
SEARCHED      (IPC)

G06F
G16H

The present search report has been drawn up for all claims

| Place of search | Date of completion of the search | Examiner |
|---|---|---|
| Munich | 26 April 2018 | Yotova, Polina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 20 7148

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2018

| Patent document cited in search report | Publication date | Patent family member(s) | Publication date |
|---|---|---|---|
| US 2015347629 A1 | 03-12-2015 | CN 106462620 A | 22-02-2017 |
| | | EP 3149621 A1 | 05-04-2017 |
| | | US 2015347629 A1 | 03-12-2015 |
| | | WO 2015187476 A1 | 10-12-2015 |

EPO FORM P0459

For more details about this annex : see Official Journal of the European Patent Office, No. 12/82